# EUROPEAN PATENT APPLICATION

(11) **EP 4 257 589 A1**
(43) Date of publication of application: **11.10.2023**
(21) Application number: 21902531.9
(22) Date of filing: 06.12.2021
(51) Int. Cl.: C07D 487/04, C07D 519/00, A61P 1/02, A61P 29/00

(54) **TRIAZOLO PYRAZINE COMPOUND AND USE THEREOF**

(30) Priority: 07.12.2020 CN 202011432143
(71) Applicant: Technoderma Medicines Inc., Chengdu, Sichuan 610219 (CN)
(72) Inventor: FANG, Wenkui, Jiaxing, Zhejiang 314000 (CN); LI, Guanqun, Jiaxing, Zhejiang 314000 (CN); CAI, Yuting, Jiaxing, Zhejiang 314000 (CN); PAN, Xiang, Jiaxing, Zhejiang 314000 (CN); ZHU, Wenhao, Jiaxing, Zhejiang 314000 (CN); WANG, Yang, Jiaxing, Zhejiang 314000 (CN); WANG, Zengquan, Jiaxing, Zhejiang 314000 (CN)
(74) Representative: Brann AB
(86) International application number: PCT/CN2021/135633
(87) International publication number: WO 2022/121825

(57) **Abstract**

A triazolo pyrazine compound, wherein the triazolo pyrazine compound is a compound as represented by formula (I) as follows, or a stereoisomer, a geometrical isomer, a tautomer, a hydrate, a solvate, and a pharmaceutically acceptable salt or a prodrug thereof, wherein W is selected from a substituted or unsubstituted aryl or heteroaryl, X is C or N, and R is H or any substituent group. When the triazolo pyrazine compound provided by the invention is used as a Tyk2 specific inhibitor, a more targeted drug can be provided for autoimmune inflammatory diseases driven by an IL-23/Th17 axis, and the compound can be used for treating rheumatoid arthritis, psoriasis, ankylosing spondylitis, sicca syndrome, lupus erythematosus, inflammatory bowel disease, Behcet's disease, severe COVID-19 pneumonia and other diseases more safely and effectively.

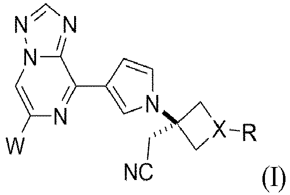

## Description

### CROSS-REFERENCE TO RELATED APPLICATIONS

This application claims the priority to a patent application No. CN202011432143.1 filed on December 07, 2020 and entitled "TRIAZOLOPYRAZINE COMPOUND AND USE THEREOF", which is incorporated herein by reference in its entirety.

### TECHNICAL FIELD

The present invention pertains to the field of small molecule compounds, and specifically relates to a triazolopyrazine compound and its use, where the triazolopyrazine compound can be used for prevention or treatment of autoimmune diseases, such as rheumatoid arthritis, inflammatory bowel disease, systemic lupus erythematosus, Sjogren's syndrome, and severe COVID-19 pneumonia, or related inflammatory skin diseases, such as psoriasis, lichen planus, vitiligo, hidradenitis suppurativa, cutaneous lupus erythematosus, or lichen sclerosus et atrophicus.

### BACKGROUND

JAK (Janus Kinase) is a family of intracellular non-receptor tyrosine protein kinases, which includes four members JAK1, JAK2, JAK3, and Tyk2. A JAK-STAT (Signal Transducer and Activator of Transcription) signaling pathway is a main pathway of intracellular signaling stimulated after binding of inflammatory cytokines and receptors, and mainly mediates various innate and acquired immune responses against attacks of various internal and external pathogenic factors including viruses, mycobacteria, and other microorganisms, thereby maintaining homeostasis and immune balance of human body. However, due to a certain unknown reason, an immune response stimulated by a pathogenic factor persists and aggravates and further develops into a chronic inflammatory disease even after the pathogenic factor disappears. As revealed by many evidences, excessive activation of the JAK-STAT signaling pathway is indispensable in pathogenesis of many diseases, especially autoimmune diseases, such as rheumatoid arthritis, lupus erythematosus, inflammatory bowel disease, psoriasis, alopecia areata, and vitiligo, and allergic diseases, such as asthma, allergic rhinitis, allergic conjunctivitis, atopic dermatitis, and eczema. Tyk2 plays an indispensable driving role in the pathogenesis of autoimmune diseases due to its specificity in mediating signaling of autoimmune-related cytokines such as IL-12, IL-23, IFN-α, and β. Therefore, efficient small molecules used to inhibit Tyk2 kinase activity can block signaling pathways mediated by cytokines involved in inflammatory response, thereby controlling inflammation, and effectively treating autoimmune- related diseases.

During onset of different inflammatory diseases, T cells differentiate in different directions with the assist of antigen presenting cells such as monocytes based on different inflammatory inducing factors such as viral or bacterial infections, autoantigens, and allergens, to form Th1, Th2, Th17 and other T cell subsets. These T cells, monocytes, and tissue cells correspondingly produce different cytokines in response to different stimulating factors, for example, Th1 cytokines (including IFN, IL-2, and IL-12) related to acute inflammations caused by viral infections, allergy-related Th2 cytokines such as IL-4, IL-5, and IL-13, and autoimmunity-related Th17 cytokines such as IL-17, IL-12, IL-21, IL-22, and IL-23. After binding to receptors on cell surfaces, these cytokines transmit inflammatory signals through JAK in the cells to promote a pathological process of the disease.

Immune responses mediated by cytokines are firstly essential for normal responses of the human immune system to various pathogenic factors in the environment and human body. When these immune responses persist after elimination of the pathogenic factors, chronic inflammatory diseases are caused, and thus inhibition of cytokines and their signaling pathways has become a focus in the development of new therapies for inflammatory diseases, and increasing studies have shown that selective inhibition of the activity of JAK subtypes helps implement both high efficiency and safety. As for autoimmune diseases, Tyk2 is responsible for signaling of IL-23/Th17 axis, presence of Th17 cells and Th17-related cytokines is crucial to autoimmunity, IL-23 is essential for induced differentiation of Th17 cells, IL-12, IFN-α and IFN-β are also related to pathogenesis of autoimmune diseases, and signaling of these important cytokines depends on Tyk2. Therefore, the inhibition of Tyk2 rather than other JAK subtypes is more beneficial to autoimmune-related diseases, which aids in efficiently and safely treating such autoimmune inflammatory diseases.

### SUMMARY

The present invention aims to provide a highly potent Tyk2-specific inhibitor, to provide a better targeted treatment for autoimmune inflammatory diseases stimulated by IL-23/Th17 axis. The Tyk2-specific inhibitor may be more suitable for treatment of rheumatoid arthritis, psoriasis, ankylosing spondylitis, Sjogren's syndrome, lupus erythematosus, Behcet's disease, and severe COVID-19 pneumonia. The highly selective Tyk2-specific inhibitor can not only prevent hematopoietic inhibition and coagulation abnormality caused by inhibiting JAK2, but also prevent viral infections that are likely caused by decrease in interferon responses due to inhibition of JAK1. In addition, because Tyk2 mediates innate immune functions against viruses and bacilli, the selection of a Tyk2 inhibitor suitable for topical application can help avoid risks of systemic treatments, for example, infections, and the Tyk2-specific inhibitor is of great significance for autoimmune inflammatory skin diseases dominated by mild to moderate symptoms.

In addition, active centers (JH1 domains) of ATP-binding parts of four members of the JAK kinase family are of high homology conservation. For a long time, various drug development companies have attempted to find highly selective and specific small molecule inhibitors, especially specific inhibitors for an active center of Tyk2 kinase, but have not made any desired breakthroughs so far. The specific ATP-competitive Tyk2 inhibitor provided in the present invention is firstly discovered, has extremely high inhibition specificity to Tyk2, and thus is of great significance for the development of Tyk2 inhibitors for the treatment of various autoimmune diseases and inflammatory skin diseases.

To achieve the foregoing objective, an aspect of the present invention provides a triazolopyrazine compound which is a compound represented by the following formula I, or stereoisomer, geometric isomer, tautomer, hydrate, solvate, and pharmaceutically acceptable salt or prodrug thereof: 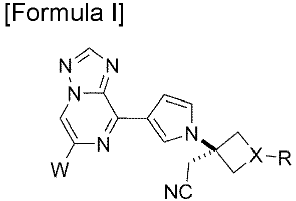
where W is selected from a substituted or an unsubstituted aryl group or heteroaryl group;
X is C or N; and
R is H or any substituent group.

In an embodiment, W is a substituted or an unsubstituted C5-C6 monocyclic group or C8-C10 polycyclic group.

In an embodiment, W is one selected from the following groups:

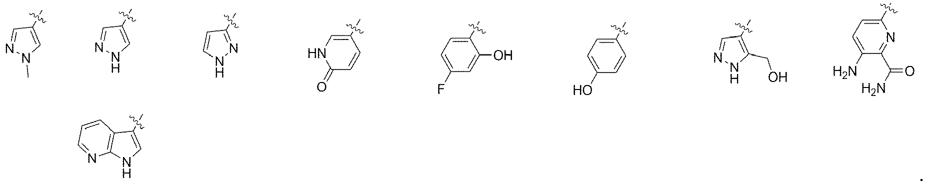

In another embodiment, R is selected from H, halogen, a cyano group, an amino group, a C1-C5 alkyl group, a C1-C5 alkoxy group, a cyanoalkyl group, a fluoroalkyl group, a hydroxyalkyl group, a C1-C5 alkyl group substituted with a benzene rang, a C3-C6 cycloalkyl group, a C3-C8 halogenated cycloalkyl group, a C3-C6 heterocycloalkyl group, a spirocycloalkyl group, a formyl group, a C5-C10 aryl group, a C5-C10 heteroaryl group, a heteroalicyclic group, an amido group, or a sulfamine group.

In another embodiment, when X is C, R is selected from halogen, a C1-C5 alkyl group, a C1-C5 haloalkyl group, a cyano group, an amino group, a C3-C6 cycloalkyl group, a C3-C6 heterocycloalkyl group, a C5-C10 aryl group, a C5-C10 heteroaryl group, an amido group, or a sulfamine group.

In another embodiment, when X is N, R is selected from a C1-C5 alkyl group, a C1-C5 haloalkyl group, a cyano group, an amino group, a C3-C6 cycloalkyl group, an amido group, or a sulfamine group.

In another embodiment, the halogen is F, Cl, or Br, the alkyl group is a methyl group, an ethyl group, a propyl group, or an isopropyl group, and/or the cycloalkyl group is a cyclopropyl group, a cyclobutyl group, or a cyclopropylmethyl group.

Another aspect of the present invention further provides use of the foregoing triazolopyrazine compound in preparation of a medicament for prevention or treatment of an autoimmune disease and a related inflammatory skin disease, where pathogenesis of all these diseases is related to disorder of JAK kinase pathway signaling. Preferably, a route of administration of the medicament includes but is not limited to oral administration, topical application, or nebulization administration.

In an embodiment, the autoimmune disease is selected from at least one of rheumatoid arthritis, inflammatory bowel disease, systemic lupus erythematosus, Sjogren's syndrome, dermatomyositis, ankylosing spondylitis, multiple sclerosis, Behcet's disease, severe COVID-19 pneumonia, Reiter's syndrome, and uveitis.

In another embodiment, the related inflammatory skin disease is selected from at least one of psoriasis, autoimmune-related vasculitis, scleroderma, dermatomyositis, acrodermatitis enteropathica, hidradenitis suppurativa, lichen planus, vitiligo, cutaneous lupus erythematosus, and lichen sclerosus et atrophicus.

Functions and effects of the present invention:

As the Tyk2-specific inhibitor, the triazolopyrazine compound provided in the present invention can provide a better targeted treatment for autoimmune inflammatory diseases stimulated by IL-23/Th17 axis. The triazolopyrazine compound is more suitable for treatment of rheumatoid arthritis, psoriasis, ankylosing spondylitis, Sjogren's syndrome, lupus erythematosus, Behcet's disease, and the like, and can not only prevent hematopoietic inhibition and coagulation abnormality caused by inhibiting JAK2, but also prevent viral infections that are likely caused by decrease in interferon responses due to inhibition of JAK1. In addition, the Tyk2 inhibitor provided in the present invention can help avoid risks of systemic treatments, and is of great significance for autoimmune inflammatory skin diseases dominated by mild to moderate symptoms.

### DETAILED DESCRIPTION

The detailed description of the present invention will be described in detail hereafter. It should be understood that, the detailed description described here are only intended to illustrate and explain the present invention, rather than limiting the present invention.

The endpoints and any value of ranges disclosed herein are not limited to precise ranges or values, and these ranges or values should be understood to include values close to these ranges or values. For numerical ranges, combination can be made among the endpoint values of each range, between the endpoint values of each range and individual point values, and among individual point values to obtain one or more new numerical ranges, and these numerical ranges should be regarded as being specifically disclosed herein.

Before detailed description of the present invention, it should be understood that the terms used herein are intended to describe a specific embodiment other than limit the scope of the present invention, where the scope of the present invention is limited only by the appended claims. To more completely understand the present invention described herein, the following terms are used, and the definitions of terms are shown below. Unless otherwise defined, all technical and scientific terms used herein have the same meanings as those understood by persons of ordinary skills in the art to which the present invention belongs.

An aspect of the present invention provides a triazolopyrazine compound which is a compound represented by the following formula I, or stereoisomer, geometric isomer, tautomer, hydrate, solvate, and pharmaceutically acceptable salt or prodrug thereof: 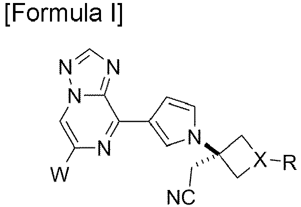
where W is selected from a substituted or an unsubstituted aryl group or heteroaryl group;
X is C or N; and
R is H or any substituent group.

In the present invention, substituted or unsubstituted aryl groups or heteroaryl groups may each independently include 1 or 2 monocyclic groups or polycyclic groups and have 5 to 10 (namely, C5-C10, for example, 5, 6, 7, 8, 9 or 10) ring atoms. In a preferable embodiment, W may be a substituted or an unsubstituted C5-C6 monocyclic group or C8-C10 polycyclic group. In a more preferable embodiment, W may include but not limited to a benzene ring, a pyridine ring, or a pyrrole ring. In addition, for the heteroaryl group, heteroatoms therein can be N, O, S or the like known to persons skilled in the art, or preferably N atoms, and the number of heteroatoms can be, for example, 1, 2, or 3.

Further, in a more preferable embodiment of the present invention, W may be selected from any one of the following groups:

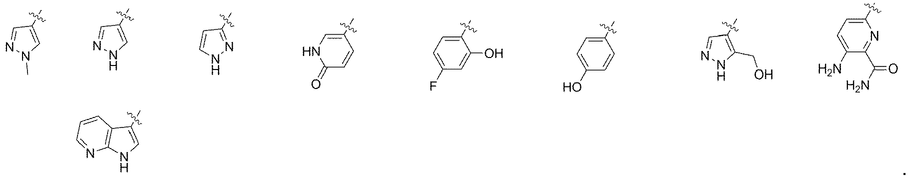

As used herein, the term "substituent" or "substituted" means that any one or more hydrogen atoms on a specified atom or group are replaced with a part selected from a specified group. In a preferable embodiment, halogen, an alkyl group, a cyano group, an amino group, an amido group, a cycloalkyl group, a heterocycloalkyl group, an aryl group, or a heteroaryl group may be used as the substituent. Further, in another preferable embodiment, halogen, an alkyl group, a cyano group, an amino group, an amido group, or a cycloalkyl group may be used as the substituent. Further, in another preferable embodiment, the halogen is F, Cl, or Br, the alkyl group is a methyl group, an ethyl group, a propyl group, or isopropyl group, and/or the cycloalkyl group is a cyclopropyl group, a cyclobutyl group, or a cyclopropylmethyl group. It should be understood that methods of making conventional substitutions to molecular structures in the present invention without affecting overall properties of the molecules should be well known to persons skilled in the art.

In addition, an R group in a right part of the triazolopyrazine compound in the present invention may be a common substituent group in the art. This is not specially limited. In a preferable embodiment, R may be selected from H, halogen, a cyano group, an amino group, an alkyl group, an alkoxy group, a cyanoalkyl group, a haloalkyl group, a hydroxyalkyl group, an alkyl group substituted with benzene, a cycloalkyl group, a halogenated cycloalkyl group, a heterocycloalkyl group, a spirocycloalkyl group, a formyl group, an aryl group, a heteroaryl group, a heteroalicyclic group, an amido group, or a sulfamine group. More preferably, when X is C, R may be selected from halogen, a C1-C5 alkyl group, a C1-C5 haloalkyl group, a cyano group, an amino group, a C3-C6 cycloalkyl group, a C3-C6 heterocycloalkyl group, a C5-C10 aryl group, a C5-C10 heteroaryl group, an amido group, or a sulfamine group; or when X is N, R may be selected from a C1-C5 alkyl group, a C1-C5 haloalkyl group, a cyano group, an amino group, a C3-C6 cycloalkyl group, an amido group, or a sulfamine group.

The term "pharmaceutically acceptable" used herein means that a substance does not affect biological activity or properties of a compound in the present invention, and is relatively non-toxic, that is, the substance can be administered to an individual without causing an adverse biological reaction or having interaction in a harmful manner with any constituent contained in the composition. In the present invention, "pharmaceutically acceptable salt" may include inorganic salt and organic salt, where the organic salt may include but not limited to ammonium, lithium, sodium, potassium, cesium, calcium, magnesium, copper, aluminum, zinc, barium or quaternary ammonium salt, and the inorganic salt may include but not limited to arginine, tert-butylamine, dimethylamine, diethanolamine, ethanolamine, ethylenediamine, imidazole, lysine, methylamine, pyridine, picolyl ester, piperazine, triethylamine, triethanolamine, trimethylamine, or urea salt.

Another aspect of the present invention provides use of the triazolopyrazine compound in inhibition of JAK kinase, especially, for use as Tyk2-specific inhibitor.

Another aspect of the present invention further provides use of the foregoing triazolopyrazine compound in preparation of a medicament for prevention or treatment of an autoimmune disease and a related inflammatory skin disease. As revealed in studies, pathogenesis of all these diseases is related to disorder of JAK kinase pathway signaling.

The term "treatment" used herein refers to any administration of a therapeutic agent according to a therapeutic regimen, where the therapeutic regimen achieves desired effects, that is, effects of partially or completely relieving, improving, remitting, inhibiting, delaying, and reducing severity and/or incidence of one or more symptoms or conditions of a particular disease, disorder, and/or condition; and in some embodiments, administration of a therapeutic agent according to a therapeutic regimen is associated with achievement of desired effects. Such treatment may be prescribed for a subject who do not have a relevant disease, disorder, and/or symptom, and/or a subject having only an early sign of the disease, disorder and/or condition. Alternatively or additionally, such treatment may be prescribed for a subject having one or more established signs of the relevant disease, disorder, and/or condition. In some embodiments, the treatment may be prescribed for a subject who has been diagnosed with the relevant disease, disorder, and/or condition. In some embodiments, the treatment may be prescribed for a subject for whom one or more predisposing factors have been identified, where the predisposing factors are statistically associated with an increased risk of developing the relevant disease, disorder, and/or condition.

Based on the present invention, the medicament prepared for the foregoing use may include an effective amount of triazolopyrazine compound in the present invention, and a pharmaceutically acceptable excipient, carrier, or diluent.

The term "effective dose", "therapeutically effective dose", or "pharmaceutically effective dose" used herein refers to a dose of a therapeutic agent that confers a therapeutic effect to a treated subject at an appropriate benefit-risk ratio applicable to any medication. Such a therapeutic effect may be objective (that is, the therapeutic effect may be measured through a specific test or marker) or subjective (that is, the effect is indicated or felt by the subject). In some embodiments, the "therapeutically effective dose" refers to a dose of a therapeutic agent or a composition for effectively treating, improving, or preventing (for example, delaying onset) of a related disease or symptom, and/or conferring a detectable therapeutic or prophylactic effect by improving a symptom associated with a disease, preventing or delaying the onset of the disease, and/or also reducing severity or frequency of the symptom.

Persons skilled in the art should understand that the therapeutically effective dose of the triazolopyrazine compound to be administered depends on the following items: nature and severity of the subject and disease, a physical condition of the subject, a treatment regimen (for example, with or without a second therapeutic agent), and a selected route of administration; and an appropriate dose can be readily determined by persons skilled in the art. Additionally, the optimal dose and interval of the medicament for individuals depends on nature and severity of a treated condition, a form, a route, and a location of administration, and age and condition of a specific treated subject, and an appropriate dose to be administered is finally at the discretion of the physician. The dose can be repeatedly used in multiple times as needed. If a side effect occurs, the dose and/or frequency can be changed or reduced based on normal clinical practice.

In the present invention, the "pharmaceutically acceptable excipient, carrier, or diluent" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, emulsifier, or the like approved for use in humans or livestock by a relevant regulatory authority.

Based on the present invention, further, the medicament prepared for the foregoing use may further comprise a formulation for prevention or treatment of an autoimmune disease and an autoimmune-related inflammatory skin disease as another effective constituent in addition to the triazolopyrazine compound in the present invention as an effective constituent. Instances of such formulations include, but are not limited to, vitamin D derivatives, vitamin A derivatives, glucocorticoid, calcineurin inhibitor, or non-steroidal anti-inflammatory drugs. When the medicament contains a plurality of effective constituents, the effective constituents may be administered simultaneously, sequentially, or separately at the discretion of the physician.

In addition, the triazolopyrazine compound in the present invention can be administered to a patient by a variety of routes, for example, the oral, transdermal, subcutaneous, intranasal, intravenous, intramuscular, intrathecal, regional, or topical (for example, mucosa) route. The most appropriate route of administration in any given situation depends on the subject and the nature and severity of the disease, physical conditions of the subject, and the like. In an embodiment, the triazolopyrazine compound in the present invention may be administered intravenously. In another embodiment, the triazolopyrazine compound in the present invention may be administered orally. Correspondingly, based on different routes of administration, the medicament in the present invention can be prepared into different dosage forms. For example, in an embodiment, the medicament may be prepared as a tablet, a capsule, a pill, a granule, an atomizer, a spray, or an injection.

After studies, the inventor found that the triazolopyrazine compound and its preparation in the present invention can have a good potency for prevention or treatment of JAK-related autoimmune disease and a related inflammatory skin disease. Specifically, the autoimmune disease is selected from rheumatoid arthritis, inflammatory bowel disease, systemic lupus erythematosus, Sjogren's syndrome, dermatomyositis, ankylosing spondylitis, multiple sclerosis, Behcet's disease, severe COVID-19 pneumonia, Reiter's syndrome, uveitis, and the like; and the related inflammatory skin disease is selected from psoriasis, autoimmune-related vasculitis, scleroderma, dermatomyositis, acrodermatitis enteropathica, hidradenitis suppurativa, lichen planus, vitiligo, cutaneous lupus erythematosus, lichen sclerosus et atrophicus, and the like.

The following examples describe a potency of the specific compound in the present invention in detail.

### Example

### Example 1: General Method for Synthesizing Compound 1 (TDM-181020)

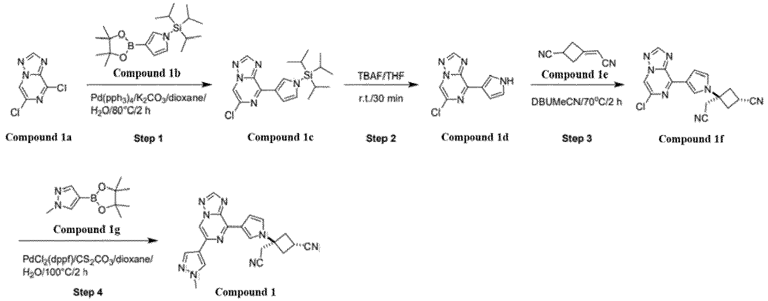

### Step 1: Preparation of Compound 1c (6-chloro-8-(1-(triisopropylsilyl)-1H-pyrrol-3-yl)-[1,2,4]triazolo[1,5-a]pyrazine)

**Compound 1a** (500 mg, 2.65 mmol), **compound 1b** (924 mg, 2.65 mmol), tetrakis(triphenylphosphine)palladium (214 mg, 0.19 mmol), potassium carbonate (732.5 mg, 5.3 mmol), dioxane (30 mL), and water (30 mL) were added into a three-necked flask. Nitrogen displacement was employed several times, and then the mixture was heated to 80°C and stirred for 60 minutes until the reaction ended under monitoring of LCMS. Post-treatment: The reaction solution was concentrated under reduced pressure, and the residue was purified by silica gel chromatography [eluent: ethyl acetate/petroleum ether = 0%-50%] to obtain yellow solid target compounds **(compound 1c,** 252.5 mg; and **compound 1d,** 95.7 mg), LCMS [M+1]⁺ = 220,376.

### Step 2: Preparation of Compound 1d (6-chloro-8-(1H-pyrrol-3-yl)-[1,2,4] triazolo[1,5-a]pyrazine)

Tetrabutylammonium fluoride (2 mL, 2.01 mmol) was added into a solution of **compound 1c** (252.5 mg, 0.67 mmol) in tetrahydrofuran (10 mL). The reaction solution was stirred for 30 minutes at room temperature until the reaction ended under monitoring of LCMS. Post-treatment: The reaction solution was concentrated under reduced pressure, and the residue was purified by silica gel chromatography [eluent: ethyl acetate/petroleum ether = 0%-50%] to obtain a yellow solid target compound **(compound 1d,** 123 mg, yield: 83.3%), LCMS [M+1]⁺ = 220.

### Step 3: Preparation of Compound 1f ((1r,3r)-3-(3-(6-chloro-[1,2,4] triazolo[1,5-a]pyrazin-8-yl)-1H-pyrrole-1-yl)-3-(cyanomethyl)cyclobutane-1-carbonitrile)

**Compound 1e** (161 mg, 1.36 mmol) and DBU (94 mg, 0.62 mmol) were added into a solution of **compound 1d** (198.7 mg, 0.91 mmol) in acetonitrile (20 mL), and the reaction solution was heated to 70°C and stirred for 2 hours until the reaction ended under monitoring of LCMS. Post-treatment: The reaction solution was concentrated under reduced pressure, and the residue was purified by silica gel chromatography [eluent: ethyl acetate/petroleum ether = 0%-50%] to obtain an off-white solid target compound (**compound 1f**, 95.2 mg, yield: 62.3%), LCMS [M+1]⁺ = 338.

¹H NMR (400 MHz, DMSO) δ 9.17 (s, 1H), 8.77 (s, 1H), 8.37 - 8.18 (m, 1H), 7.22 (dd, J = 3.0, 2.4 Hz, 1H), 7.14 (dd, J = 3.1, 1.6 Hz, 1H), 3.56 - 3.45 (m, 1H), 3.43 (s, 2H), 3.16 - 3.08 (m, 2H), 2.94 - 2.86 (m, 2H).

### Step 4: Preparation of Compound 1 ((1r,3r)-3-(cyanomethyl)-3-(3-(6-(1-methyl-1H-pyrazol-4-yl)-[1,2,4]triazolo[1,5-a]pyrazin-8-yl)-1H-pyrrol-1-yl)cyclobutane-1-carbonitrile)

**Compound 1f** (95.2 mg, 0.28 mmol), **compound 1g** (58.6 mg, 0.28 mmol), 1,1'-bis(diphenylphosphino)ferrocene-dichloropalladium (22 mg, 0.03 mmol), cesium carbonate (184 mg, 0.56 mmol), dioxane (12 mL), and water (2 mL) were added into a three-necked flask. Argon displacement was employed for the reaction solution several times, heated to 100°C and stirred for 2 hours until the reaction ended under monitoring of LCMS. Post-treatment: A reaction solution was concentrated under reduced pressure, ethyl acetate (50 mL) and water (50 mL) were added into a residue for layered extraction, an organic phase was washed once with saturated brine, dried over anhydrous sodium sulfate, and filtered via suction, the filtrate was concentrated to dryness, and the residue was purified by silica gel chromatography [eluent: (D/M = 10:1)/DCM = 0%-40%]. A resulting crude product was then purified to obtain a yellow solid target compound (**compound 1**, 31.6 mg, yield: 29.22%), LCMS [M+1]⁺ = 384.

¹H NMR (400 MHz, DMSO) δ 9.16 (s, 1H), 8.67 (s, 1H), 8.39 (s, 1H), 8.35-8.29 (m, 1H), 8.16 (d, J = 0.6 Hz, 1H), 7.27 (dd, J = 3.0, 1.6 Hz, 1H), 7.23 - 7.18 (m, 1H), 3.93 (s, 3H), 3.57 - 3.47 (m, 1H), 3.43 (s, 2H), 3.19 - 3.10 (m, 2H), 2.94 - 2.85 (m, 2H).

### Example 2: General Method for Synthesizing Compound 2 (TDM-181021)

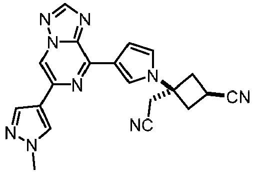

### Step 1: Preparation of Compound 2 ((1s,3s)-3-(cyanomethyl)-3-(3-(6-(1-methyl-1H-pyrazol-4-yl)-[1,2,4]triazolo[1,5-a]pyrazin-8-yl)-1H-pyrrol-1-yl)cyclobutane-1-carbonitrile)

Compound 2 (white solid, 38.4 mg, yield: 26.7%) was prepared in a method similar to that in Example 1.

¹H NMR (400 MHz, DMSO) δ 9.16 (s, 1H), 8.67 (s, 1H), 8.39 (s, 1H), 8.27-8.21 (m, 1H), 8.16 (s, 1H), 7.25 (dd, J = 3.0, 1.6 Hz, 1H), 7.16 - 7.09 (m, 1H), 3.93 (s, 3H), 3.62 (p, J = 8.8 Hz, 1H), 3.45 (s, 2H), 3.05 - 2.85 (m, 4H). LCMS [M+1]⁺ = 384.2.

### Test Example 1: Detection of Enzyme Activity Inhibition of Small Molecule Inhibitors of JAK Kinases

### Test Scheme

### 1. Reagent Preparation

Kinase reaction buffer: Kinase reaction buffer was prepared with the following constituents: 50 mM HEPES, pH 7.5, 1 mM EGTA, 10 mM MgCl₂, 2 mM DTT, and 0.01% Tween 20. 1 × test buffer: The test buffer was prepared, and diluted 10-fold with deionized water (deionized water: test buffer = 9:1) to obtain the 1 × test buffer. 4 × kinase solution: JAK kinase was diluted 4-fold with the kinase reaction buffer to a final concentration (JAK1: 80 nM, JAK2/JAK3/Tyk2: 4 nM). 4 × substrate solution: ULight^{™}-JAK (Tyr1023) substrate was diluted with the kinase reaction buffer to 200 nM (final concentration: 50 nM). 4 × ATP solution: ATP was diluted 4-fold with the kinase reaction buffer to a final concentration (JAK1: 160 µM, JAK2/JAK3/Tyk2: 40 µM). 4 × test compound solution: The test compound was dissolved with DMSO to prepare 10 mM stock solutions, and diluted 3-fold serially to required concentrations, 10 concentrations were set for each compound, and final concentrations of the test compound ranged from 10 µM to 0.5 nM. 4 × enzyme reaction stop solution: 1 × test buffer was used dissolve EDTA to 40 mM (final concentration of EDTA: 10 mM). 4 × test antibody solution: Eu-labeled test antibody (anti-phosphotyrosine (PT66)) was diluted with 1 × test buffer to 8 nM (final concentration of antibody: 2 nM).

### 2. Test Procedure

2.5 µL of 4 × kinase solution and 2.5 µL of 4 × test compound solutions diluted to different concentrations were added into a 384-well microwell plate sequentially, 2 duplicate wells were disposed for each concentration, and a blank control group and a negative control group (DMSO group) were also provided for the enzyme solutions. The 384-well plate was oscillated to mix the enzyme and compound uniformly, centrifuged at 1000 rpm for 1 minute, and incubated at room temperature for 60 minutes. 2.5 µL of 4 × substrate solution was added into the 384-well plate, and centrifuged at 1000 rpm for 1 minute. 2.5 µL of 4 × ATP solution was added into the 384-well plate, and centrifuged at 1000 rpm for 1 minute to start the enzyme reaction. JAK1 reacted at room temperature for 2 hours, and JAK2, JAK3, or Tyk2 reacted at room temperature for 1 hour. Final concentrations of constituents in the JAK1 reaction were: JAK1: 20 nM; substrate: 50 nM; and ATP: 40 µM. Final concentrations of the test compounds ranged from 10 µM to 0.5 nM. Final concentrations of constituents in the JAK2, JAK3, or Tyk2 reaction were: JAK2: 1 nM; substrate: 50 nM; and ATP: 10 µM. Final concentrations of the test compounds ranged from 10 µM to 0.5 nM. After the enzyme reaction ended, 5 µL of 4 × enzyme reaction stop solution was added into each well of the 384-well plate, centrifuged at 1000 rpm for 1 minute, and incubated at room temperature for 5 minutes. 5 µL of 4 × test antibody solution (final concentration of the test antibody was 2 nM) was added into each well of the 384-well plate, centrifuged at 1000 rpm for 1 minute, and incubated at room temperature for 1 hour. After incubation of the antibody, a signal value of each well was measured via Envision plate reader.

### 3. Data Analysis

Assuming that an inhibition rate of the blank control group of the enzyme solution was 100% and an inhibition rate of the negative control group (DMSO group) was 0%, an inhibition rate corresponding to each concentration was calculated. GraphPad Prism was used to perform nonlinear regression analysis on log values of concentrations of the test compounds and the corresponding inhibition rates to obtain half-maximum inhibitory concentrations (IC₅₀) of the test compounds. The test results measured for the compounds in Examples 1 to 10 are listed in Table 1 below.

**Table 1**

| No. | Tyk2/µM | JAK1/µM | JAK2/µM | JAK3/µM |
|---|---|---|---|---|
| TDM-181020 | 0.111 | >5 | 2.420 | >5 |
| TDM-181021 | 0.014 | 1.208 | 0.138 | >5 |

It could be seen from the results in Table 1 that enzyme activity data of the compounds in this application were excellent, and the half-maximum inhibitory concentrations measured for the above specific compounds were low, especially a compound targeting Tyk2. Therefore, the above tests revealed that the small molecular compounds in this application were compounds with high specificity to JAK family and excellent enzymatic activity, and especially could be used as Tyk2-specific inhibitors.

The preferred embodiments of the present invention have been described in detail above. However, the present invention is not limited to the specific details in the above embodiments. Within the scope of the technical concept of the present invention, many simple modifications can be made to the technical solution of the present invention, all of which fall within the claimed scope of the present invention.

Furthermore, it should be noted that various specific technical features described in the above specific embodiments can be combined in any suitable way without contradiction. In order to avoid unnecessary repetition, the present invention will not explain various possible combinations separately.

Furthermore, any combination can be made among various embodiments of the present invention, as long as it does not violate the idea of the present invention, it should also be regarded as the disclosure of the present invention.

## Claims

1. A triazolopyrazine compound which is a compound represented by formula I, or stereoisomer, geometric isomer, tautomer, hydrate, solvate, and pharmaceutically acceptable salt or prodrug thereof: 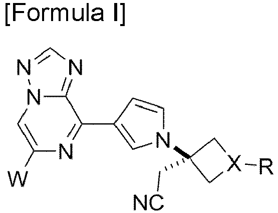
wherein W is selected from a substituted or an unsubstituted aryl group or heteroaryl group;
X is C or N; and
R is H or any substituent group.

2. The triazolopyrazine compound according to claim 1, **characterized in that** W is a substituted or an unsubstituted C5-C6 monocyclic group or C8-C10 polycyclic group.

3. The triazolopyrazine compound according to claim 2, **characterized in that** W is one selected from the following groups:

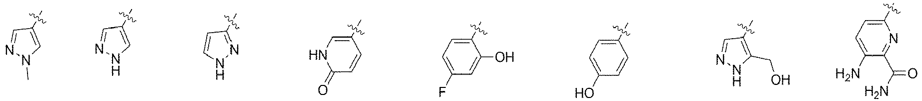

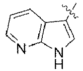

4. The triazolopyrazine compound according to claim 1, **characterized in that** R is selected from H, halogen, a cyano group, an amino group, an alkyl group, an alkoxy group, a cyanoalkyl group, a haloalkyl group, a hydroxyalkyl group, an alkyl group substituted with benzene, a cycloalkyl group, a halogenated cycloalkyl group, a heterocycloalkyl group, a spirocycloalkyl group, a formyl group, an aryl group, a heteroaryl group, a heteroalicyclic group, an amido group, or a sulfamine group.

5. The triazolopyrazine compound according to claim 4, **characterized in that** when X is C, R is selected from halogen, a C1-C5 alkyl group, a C1-C5 haloalkyl group, a cyano group, an amino group, a C3-C6 cycloalkyl group, a C3-C6 heterocycloalkyl group, a C5-C10 aryl group, a C5-C10 heteroaryl group, an amido group, or a sulfamine group.

6. The triazolopyrazine compound according to claim 4, **characterized in that** when X is N, R is selected from a C1-C5 alkyl group, a C1-C5 haloalkyl group, a cyano group, an amino group, a C3-C6 cycloalkyl group, an amido group, or a sulfamine group.

7. The triazolopyrazine compound according to claim 5 or 6, **characterized in that** the halogen is F, Cl, or Br, the alkyl group is a methyl group, an ethyl group, a propyl group, or an isopropyl group, and/or the cycloalkyl group is a cyclopropyl group, a cyclobutyl group, or a cyclopropylmethyl group.

8. Use of the triazolopyrazine compound according to any one of claims 1 to 7 in preparation of a medicament for prevention or treatment of an autoimmune disease and a related inflammatory skin disease, wherein pathogenesis of all these diseases is related to disorder of JAK kinase pathway signaling.

9. The use according to claim 8, **characterized in that** the autoimmune disease is selected from at least one of rheumatoid arthritis, inflammatory bowel disease, systemic lupus erythematosus, Sjogren's syndrome, dermatomyositis, ankylosing spondylitis, multiple sclerosis, Behcet's disease, severe COVID-19 pneumonia, Reiter's syndrome, and uveitis.

10. The use according to claim 8, **characterized in that** the related inflammatory skin disease is selected from at least one of psoriasis, autoimmune-related vasculitis, scleroderma, dermatomyositis, acrodermatitis enteropathica, hidradenitis suppurativa, lichen planus, vitiligo, cutaneous lupus erythematosus, and lichen sclerosus et atrophicus.
